# EUROPEAN PATENT APPLICATION

(11) **EP 4 847 064 A1**
(43) Date of publication of application: **02.12.2026**
(21) Application number: 24919678.3
(22) Date of filing: 07.08.2024
(51) Int. Cl.: A61M 5/32

(54) **SAFE INJECTION NEEDLE HEAD**

(30) Priority: 24.01.2024 CN 202410095538
(71) Applicant: Sandstone Medical (Suzhou) Inc., Suzhou, Jiangsu 215000 (CN)
(72) Inventor: SHI, Guoping, Suzhou, Jiangsu 215000 (CN); HORSTMAN, Anthony Scott, Suzhou, Jiangsu 215000 (CN); WU, Bo, Suzhou, Jiangsu 215000 (CN)
(74) Representative: Betten & Resch
(86) International application number: PCT/CN2024/110295
(87) International publication number: WO 2025/156596

(57) **Abstract**

A safety injection needle comprises a needle hub, a first sheath, a second sheath and a spring. The rear end of the second sheath is arranged between the first sheath and the needle hub, the front end of the second sheath is provided with a second opening and extends out from the first opening. A transmission ring is arranged between the inner side of the rear port of the second sheath and the needle hub. At least one first bump is circumferentially arranged on the outer wall of the transmission ring, and at least one second bump is circumferentially arranged on the inner wall of the transmission ring. A blocking guiding surface corresponding to the first bump is formed on the inner wall of the second sheath; the blocking guiding surface includes a flat guiding segment and an inclined guiding segment, one end of the flat guiding segment is defined as an initial position, and the other end of the inclined guiding segment is defined as an end position. An axially extending guiding groove corresponding to the second bump is formed on the outer circumference of the needle hub, and a forward-facing locking surface is formed at the front end of one side of the guiding groove. The present invention can prevent secondary use, features high operational stability and a simple structure, and is convenient for assembly.

## Description

### Technical Field

The present invention relates to the field of medical supplies, and specifically discloses a disposable safety injection needle.

### Background Art

Diabetes is a metabolic disease characterized by elevated blood glucose. There is currently no radical cure for diabetes, but insulin injection can effectively control the disease condition.

A variety of insulin injection tools are available. The injection needle of the present invention is an injection tool matched with an insulin pen. The insulin pen can store a prescribed volume of insulin liquid medicine and is reusable, while the injection needle is a disposable needle equipped with a safety protection device.

A representative conventional structure can be found in Chinese Patent Application No. CN116942966A, which discloses an invention titled *Disposable Injection Needle*. The injection needle disclosed therein basically includes a needle hub, a first sheath, a second sheath, an inner core and a compression spring. A needle tube extends axially through the needle hub. The first sheath is sleeved on the needle hub and fixedly connected to the rear end thereof, and a first opening is formed at the front end of the first sheath; the needle tube of the needle hub extends out from the first opening, forming an accommodating space between the first sheath and the needle hub; The rear end of the second sheath is arranged inside the accommodating space, the front end of the second sheath is provided with a second opening and extends out from the first opening, and the second sheath is axially slidably connected relative to the first sheath. The inner core is disposed between the second sheath and the needle hub and is rotatable in the circumferential direction relative to the needle hub; The outer wall of the inner core is provided with a limiting guide groove matched with a blocking member on the inner wall of the second sheath, and the rear end of the inner core is provided with an inner core guiding portion matched with a guiding matching portion on the needle hub, and the guiding matching portion includes a flat guiding portion and an inclined guiding portion; The compression spring has one end abutting against the second sheath and the other end abutting against the rear end of the inner core. Therefore, during use, the second sheath retracts inward under external force to the accommodating space, allowing the head of the needle tube to extend out from the second opening. As the blocking member cooperates with the limiting guide groove, the inner core rotates circumferentially, so that the inner core guiding portion rotates from the flat guiding portion onto the inclined guiding portion. After the injection needle is used, the external force applied to the second sheath is removed, and the second sheath rebounds and resets under the action of the spring. At the moment the blocking member of the second sheath separates from the limiting guide groove of the inner core, the inner core guiding portion slides along the inclined guiding portion of the needle hub under the spring force until the inner core guiding portion abuts against the limiting flat surface of the needle hub. At this time, the sheath blocking surface of the inner core has rotated to a position opposite the blocking member of the second sheath, so that the second sheath can no longer retract into the accommodating space, and the head of the needle tube cannot be exposed for a second time, thereby realizing a single-use function.

Although the above prior art realizes single-use function, its operational reliability is insufficient in practical application. The inner core serves as the core component for circumferential rotation and is provided with both limiting guide grooves and an inner core guiding portion. When the second sheath retracts inward under external force during needle use, the blocking member on the second sheath abuts against the limiting guide groove to push the inner core to rotate, resulting in unsmooth movement and jamming risk. In addition, the spring abuts the second sheath at one end and the rear end of the inner core at the other end. The second sheath performs axial expansion and contraction during use while the inner core rotates, meaning both ends of the spring move. After needle use, the spring must rebound the second core to reset while simultaneously sliding the inner core guiding portion along the inclined guiding surface of the needle hub. In this process, the spring is prone to offset or incomplete reset, which may cause the inner core to fail to rotate into place and disable the single-use protection function, leading to safety accidents.

### Summary of the Invention

The present invention provides a safety injection needle to improve the reliability of single-use protection.

To achieve the above objective, the technical solution adopted by the present invention is described as follows:

A safety injection needle, comprising a needle hub, a first sheath, a second sheath and a spring; an axially extending needle tube is arranged on the needle hub; the first sheath is sleeved on the needle hub and fixedly connected to the rear end thereof, and a first opening is formed at the front end of the first sheath; the needle tube of the needle hub extends out from the first opening, and an accommodating space is formed between the first sheath and the needle hub; the rear end of the second sheath is arranged inside the accommodating space, the front end of the second sheath is provided with a second opening and extends out from the first opening, and the second sheath is axially slidably connected relative to the first sheath.

A transmission ring is arranged between the inner side of the rear port of the second sheath and the needle hub. At least one first bump is circumferentially arranged on the outer wall of the transmission ring, and at least one second bump is circumferentially arranged on the inner wall of the transmission ring.

A blocking guiding surface corresponding to the first bump is formed on the inner wall of the second sheath. The blocking guiding surface includes a flat guiding segment and an inclined guiding segment. One end of the flat guiding segment is defined as an initial position, the other end thereof is connected to one end of the inclined guiding segment, the inclined guiding segment inclines toward the front end of the second sheath, and the other end of the inclined guiding segment is defined as an end position.

An axially extending guiding groove is formed on the outer circumference of the needle hub corresponding to the second bump. The guiding groove includes a front groove segment and a rear groove segment. The front groove segment has a larger circumferential dimension than the rear groove segment. One side wall of the front groove segment and the identical side wall of the rear groove segment are transitionally connected via an inclined guiding surface, and a locking surface facing forward is provided at the front end of the other side of the front groove segment.

The spring is sleeved on the needle hub. A spring mounting surface abutting the rear end of the spring is formed on the needle hub, and the front end of the spring abuts against the transmission ring.

In the initial state before use, the first bump of the transmission ring is located at the initial position of the blocking guiding surface, and the second bump directly faces the side wall of the front groove segment close to the inclined guiding surface. During use, the second sheath moves rearward under external force, the blocking guiding surface cooperates with the first bump to push the transmission ring to move rearward, the second bump slides into the front groove segment, and the cooperation between the second bump and the inclined guiding surface drives the transmission ring to rotate circumferentially; When the second sheath moves rearward to the limit position, the first bump slides onto the inclined guiding segment, and the second bump slides to the rear end of the rear groove segment; After use, the second sheath and the transmission ring extend and reset under the spring force, the second bump slides to the front end of the front groove segment, and under the sliding cooperation between the first bump and the inclined guiding segment, the second bump rotates to a position opposite the locking surface, so the second sheath cannot retract rearward again due to the blocking of the locking surface.

In the above solution, the locking surface corresponds to the end position of the inclined guiding segment, and the circumferential width of the locking surface is greater than the circumferential width of the end position of the inclined guiding segment. After the first bump slides into the end position, re-pressing the second sheath ensures that the second bump faces the locking surface, so the second sheath is blocked by the locking surface and cannot retract rearward again.

In the above solution, a forward protruding portion is formed at the junction of the locking surface and the front groove segment to make the locking surface concave.

In the above solution, an axial transition surface is arranged between the flat guiding segment and the inclined guiding segment of the blocking guiding surface. When the second sheath moves rearward to the limit position, the first bump abuts against the axial transition surface. The transition surface serves as a limiting structure to prevent the first bump from sliding back to the flat guiding segment as the transmission ring moves forward under spring force, which would disable the anti-reuse function.

In the above solution, a circumferential annular protrusion is formed on the rear end of the needle hub, and a clamping portion is correspondingly formed on the inner wall of the rear end of the first sheath. The first sheath and the needle hub are connected via clamping fit between the clamping portion and the annular protrusion.

In the above solution, the second sheath is provided with a transparent portion. After use, when the second sheath and the transmission ring extend and reset under spring force and the first bump reaches the end position of the inclined guiding segment, the transmission ring can be seen protruding from the first opening of the first sheath through the transparent portion of the second sheath, distinguishing it from an unused state.

In the above solution, the axial lengths of the front groove segment and the inclined guiding surface are both greater than the axial length of the rear groove segment. This provides a longer stroke during the pressing trigger phase. In case of accidental pressing (without moving close to the limit position), the second sheath can rebound under spring force, allowing the entire injection needle to be reused and realizing an accidental pressing prevention function.

In the present invention, the guiding groove is disposed on the stationary needle hub, and the blocking guiding surface is arranged on the second sheath that only performs axial movement. As the core component realizing locking through circumferential rotation, the transmission ring is merely provided with the first bump and the second bump. During operation, the first bump cooperates with the blocking guiding surface and the second bump cooperates with the guiding groove in a mode where the bumps slide along stationary guiding surfaces, so that the transmission ring rotates smoothly. In addition, the rear end of the spring abuts against the fully fixed rear portion of the needle hub, enabling stable spring reset without offset and jamming-free rotation of the transmission ring, thereby delivering highly reliable and stable operation. After use, even if the second sheath is pressed again, its rearward movement drives the transmission ring to retract, and the second bump of the transmission ring abuts the locking surface to stop further rearward displacement and prevent exposure of the needle tip. Upon releasing the second sheath, the spring resets to drive the transmission ring to the end position of the inclined guiding segment of the second sheath, such that the transmission ring protrudes out of the first sheath to form a visual distinction from the unused state.

### Brief Description of the Drawings

FIG. 1 is a schematic perspective overall view of an embodiment of the present invention;
FIG. 2 is an exploded schematic view of components of an embodiment of the present invention;
FIG. 3 is a perspective view of the second sheath according to an embodiment of the present invention;
FIG. 4 is a perspective view of the first sheath according to an embodiment of the present invention;
FIG. 5 is a perspective view of the transmission ring according to an embodiment of the present invention;
FIG. 6 is a perspective view of the needle hub according to an embodiment of the present invention;
FIG. 7 is a cross-sectional view of the embodiment in the initial state before use, with the outer casing and the first sheath removed;
FIG. 8 is a radial sectional perspective view of the embodiment in the initial state before use, with the outer casing removed;
FIG. 9 is a partial sectional perspective view No.1 of the embodiment in the initial state before use, with the outer casing removed;
FIG. 10 is a partial sectional perspective view No.2 of the embodiment in the initial state before use, with the outer casing and second sheath removed;
FIG. 11 is a schematic view showing the relative position of the transmission ring and the second sheath in the initial state before use;
FIG. 12 is a schematic view showing the relative position of the transmission ring and the needle hub in the initial state before use;
FIG. 13 is a cross-sectional view of the embodiment when the second sheath moves rearward to the limit position during use, with the outer casing removed;
FIG. 14 is a partial sectional perspective view of the embodiment when the second sheath moves rearward to the limit position during use, with the outer casing removed;
FIG. 15 is a schematic view showing the relative position of the transmission ring and the second sheath when the second sheath moves rearward to the limit position during use;
FIG. 16 is a schematic view showing the relative position of the transmission ring and the needle hub when the second sheath moves rearward to the limit position during use;
FIG. 17 is a cross-sectional view of the embodiment with the second sheath reset after use, with the outer casing removed;
FIG. 18 is a partial sectional perspective view of the embodiment with the second sheath reset after use, with the outer casing removed;
FIG. 19 is a schematic view showing the relative position of the transmission ring and the second sheath with the second sheath reset after use;
FIG. 20 is a schematic view showing the relative position of the transmission ring and the needle hub with the second sheath reset after use;
FIG. 21 is a cross-sectional view of the embodiment when the second sheath is pressed again after resetting, with the outer casing removed;
FIG. 22 is a partial sectional perspective view of the embodiment when the second sheath is pressed again after resetting, with the outer casing removed;
FIG. 23 is a schematic view showing the relative position of the transmission ring and the second sheath when the second sheath is pressed again after resetting;
FIG. 24 is a schematic view showing the relative position of the transmission ring and the needle hub when the second sheath is pressed again after resetting.

In the above drawings:
1. Needle hub; 11. Needle tube; 12. Annular protrusion; 13. Guiding groove; 131. Front groove segment; 132. Rear groove segment; 133. Inclined guiding surface; 134. Locking surface; 1341. Forward protruding portion; 14. Spring mounting surface;
2. First sheath; 21. First opening; 22. Guiding rib; 23. Clamping portion;
3. Second sheath; 31. Second opening; 32. Guiding groove; 33. Blocking guiding surface; 331. Flat guiding segment; 332. Inclined guiding segment; 333. Transition surface; A. Initial position; B. End position;
4. Spring;
5. Outer casing;
6. Transmission ring; 61. First bump; 62. Second bump;
R. Accommodating space.

### Detailed Description of Embodiments

The present invention will be further described below in conjunction with the accompanying drawings and embodiments:

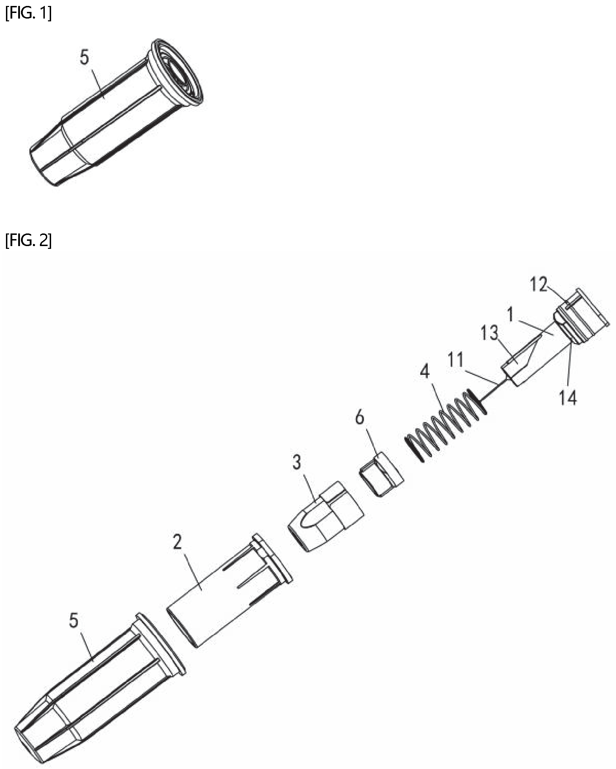

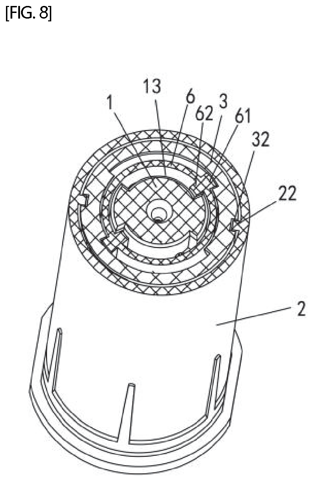

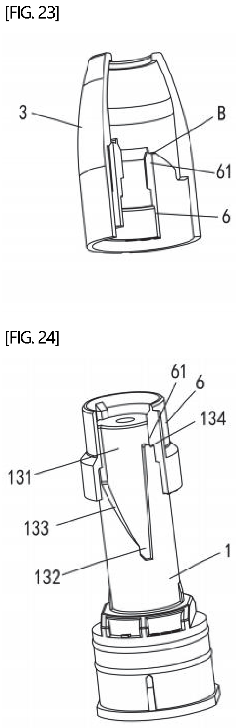

### Embodiment, as shown in FIGS. 1 to 24:

A safety injection needle comprises a needle hub 1, a first sheath 2, a second sheath 3, an outer casing 5, a spring 4 and a transmission ring 6, as shown in FIG. 2.

An axially extending needle tube 11 is arranged on the needle hub 1 as shown in FIG. 2. The direction pointed by the head of the needle tube is defined as the front, and the opposite direction is the rear.

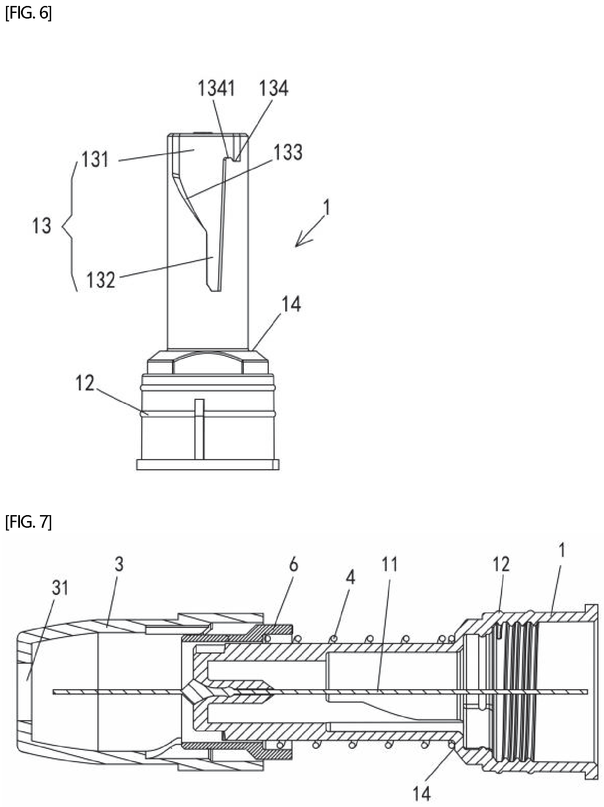

As shown in FIGS. 7, 9 and 13, the first sheath 2 is sleeved on the needle hub 1 and fixedly connected to its rear end, and a first opening 21 is formed at the front end of the first sheath 2. The needle tube 11 of the needle hub 1 extends out from the first opening 21, forming an accommodating space R between the first sheath 2 and the needle hub 1. The rear end of the second sheath 3 is arranged inside the accommodating space R, the front end of the second sheath 3 is provided with a second opening 31 and extends out from the first opening 21, and the second sheath 3 is axially slidably connected relative to the first sheath 2.

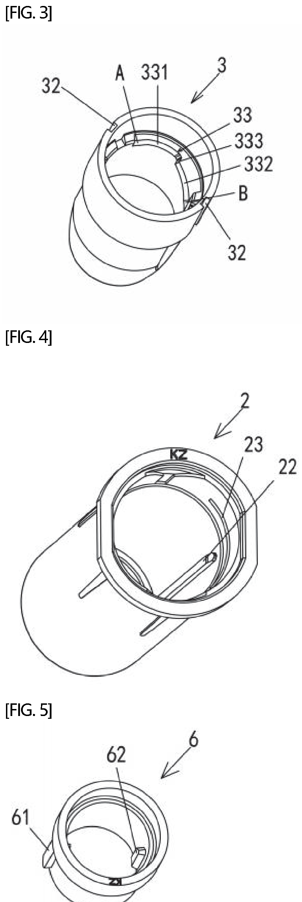

Specifically, the preferred sliding matching structure between the second sheath 3 and the first sheath 2 is shown in FIGS. 3, 4 and 8. Axially extending guiding ribs 22 are formed on the inner wall of the first sheath 2, and guiding grooves 32 corresponding to the guiding ribs 22 are formed on the outer wall of the second sheath 3. The first sheath 2 and the second sheath 3 realize sliding connection through guiding fit between the guiding ribs 22 and guiding grooves 32.

As shown in FIGS. 2, 6, 13, 17 and 21, a circumferential annular protrusion 12 is formed on the rear end of the needle hub 1, and a clamping portion 23 is correspondingly formed on the inner wall of the rear end of the first sheath 2. The first sheath 2 and the needle hub 1 are connected via clamping fit between the clamping portion 23 and the annular protrusion 12.

As shown in FIGS. 5, 7 and 8, the transmission ring 6 is arranged between the inner side of the rear port of the second sheath 3 and the needle hub 1. At least one first bump 61 (preferably two as shown in the drawings) is circumferentially arranged on the outer wall of the transmission ring 6, and at least one second bump 62 (preferably two as shown in the drawings) is circumferentially arranged on the inner wall of the transmission ring 6. The first bumps 61 protrude radially outward from the transmission ring 6, and each first bump 61 is provided with a front-facing end surface serving as a force-bearing surface. The second bumps 62 protrude radially inward from the transmission ring 6, and both side surfaces and the rear-facing end surface of each second bump 62 serve as force-bearing surfaces.

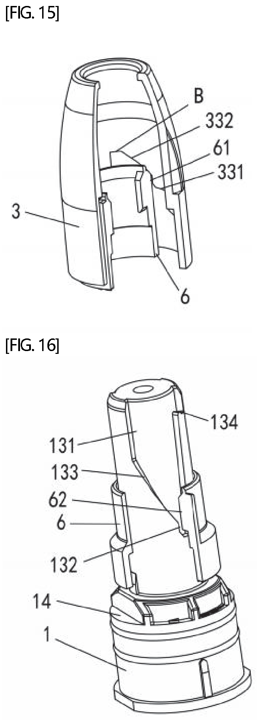

As shown in FIGS. 3 and 15, a blocking guiding surface 33 facing rearward is formed on the inner wall of the second sheath 3 corresponding to the first bumps 61. The blocking guiding surface 33 includes a flat guiding segment 331 and an inclined guiding segment 332. The projection of the flat guiding segment 331 on the axial section of the second sheath 3 is substantially a straight line perpendicular to the axial direction, while the projection of the inclined guiding segment 332 on the axial section of the second sheath 3 is an inclined line forming an angle with the axial direction. One end of the flat guiding segment 331 is defined as an initial position A, the other end is connected to one end of the inclined guiding segment 332, the inclined guiding segment 332 inclines toward the front end of the second sheath 3, and the other end of the inclined guiding segment 332 is defined as an end position B.

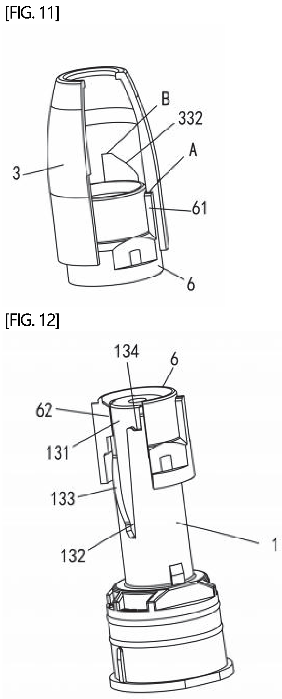

As shown in FIGS. 6 and 12, axially extending guiding grooves 13 corresponding to the second bumps 62 are formed on the outer circumference of the needle hub 1. As shown in FIG. 6, each guiding groove 13 includes a front groove segment 131 and a rear groove segment 132. The front groove segment 131 has a larger circumferential dimension than the rear groove segment 132. One side wall of the front groove segment 131 is connected to the same side wall of the rear groove segment 132 via an inclined guiding surface 133, and a locking surface 134 facing forward is formed at the front end of the other side of the front groove segment 131.

As shown in FIG. 7, the spring 4 is sleeved on the needle hub 1. A spring mounting surface 14 abutting the rear end of the spring 4 is formed on the needle hub 1, and the front end of the spring 4 abuts against the rear end of the transmission ring 6, pushing the transmission ring 6 forward via spring force.

The locking surface 134 corresponds to the end position B of the inclined guiding segment 332, and the circumferential width of the locking surface 134 is greater than the circumferential width of the end position B of the inclined guiding segment 332. After the first bump 61 slides into the end position, re-pressing the second sheath 3 ensures that the second bump 62 faces the locking surface 134, so the second sheath 3 is blocked by the locking surface 134 and cannot retract rearward again.

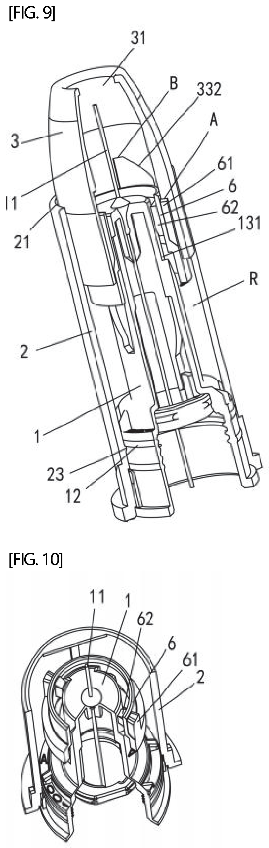

In the initial state before use, the first bump 61 of the transmission ring 6 is located at the initial position A of the blocking guiding surface 33 (as shown in FIGS. 9 and 11), and the second bump 62 directly faces the side wall of the front groove segment 131 adjacent to the inclined guiding surface 133 (as shown in FIG. 12).

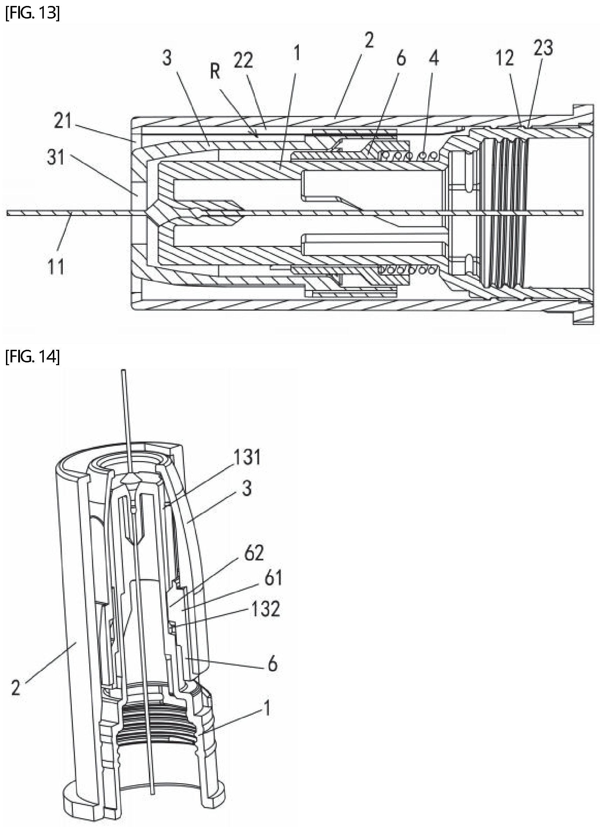

During use, the outer casing 5 is removed, and the second sheath 3 moves rearward under external force (i.e., pressing the injection needle against human skin for puncture). The blocking guiding surface 33 cooperates with the first bump 61 to push the transmission ring 6 to move rearward, the second bump 62 slides into the front groove segment 131, and the cooperation between the second bump 62 and the inclined guiding surface 133 drives the transmission ring 6 to rotate circumferentially, as shown in FIG. 16. When the second sheath 3 finally moves rearward to the limit position (as shown in FIGS. 13, 14, 15 and 16), the first bump 61 slides onto the inclined guiding segment 332 of the blocking guiding surface 33, and the second bump 62 slides to the rear end of the rear groove segment 132. At this time, the front end of the second sheath 3 is substantially flush with the front end of the first sheath 2, allowing the needle tube 11 to extend to the maximum length for puncturing human skin.

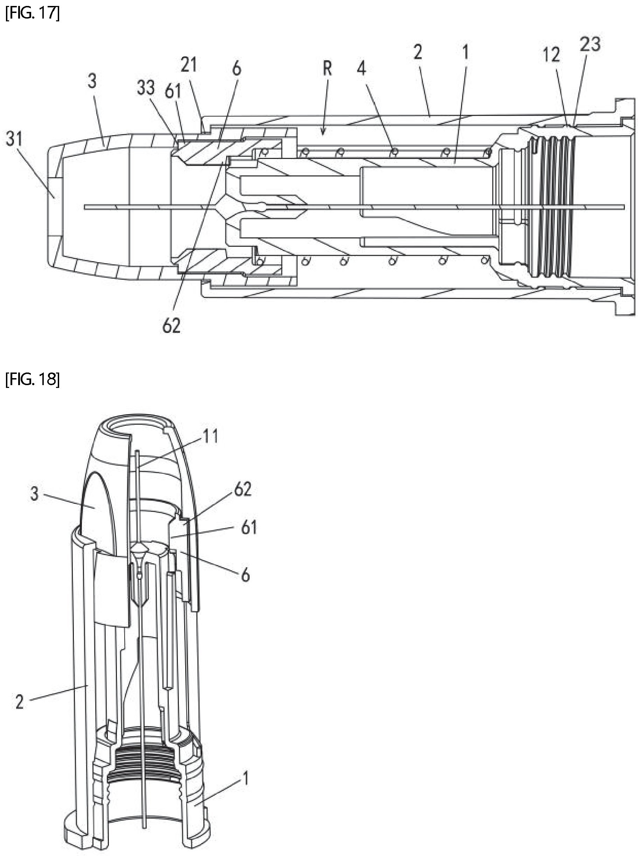

After use, the second sheath 3 and the transmission ring 6 extend forward and reset under the force of the spring 4 (i.e., pulling the needle away from human skin after injection). The second bump 62 slides to the front end of the front groove segment 131 and breaks free from the restriction of the guiding groove 13. Under the sliding cooperation between the first bump 61 and the inclined guiding segment 332, the second bump 62 of the transmission ring 6 rotates to a position opposite the locking surface 134, as shown in FIGS. 17, 18, 19 and 20. The second bump 62 of the transmission ring 6 is located in front of and opposite the locking surface 134, and the first bump 61 reaches the end position B of the inclined guiding segment 332, which is the post-use state.

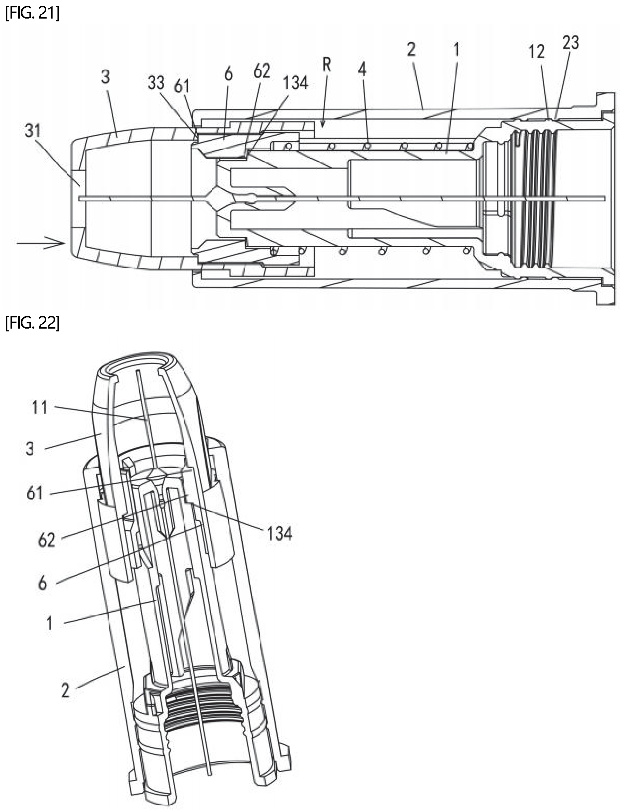

If the second sheath 3 is pressed again, the second sheath 3 will move rearward and abut against the locking surface 134, and further retraction is blocked by the locking surface 134. This state is illustrated in FIG. 21, FIG. 22, FIG. 23 and FIG. 24. The second sheath 3 permanently covers the tip of the needle tube 11 to realize the anti-reuse function.

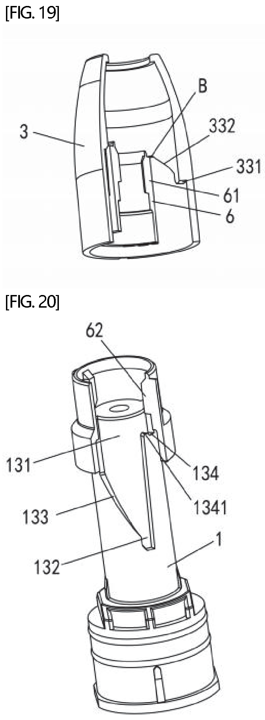

As shown in FIG. 20, a forward protruding portion 1341 is formed at the junction of the locking surface 134 and the front groove segment 131 to make the locking surface 134 concave, enabling more stable limiting and positioning of the second bump 62 by the locking surface 134.

As shown in FIG. 3, an axial transition surface 333 is arranged between the flat guiding segment 331 and the inclined guiding segment 332 of the blocking guiding surface 33. When the second sheath 3 moves rearward to the limit position, the first bump 61 abuts against the axial transition surface 333. The transition surface 333 serves as a limiting structure to prevent the first bump 61 from sliding back toward the flat guiding segment 331 as the transmission ring 6 moves forward under spring force, thus reliably realizing the anti-reuse function.

As shown in FIG. 3, the initial position A of the blocking guiding surface 33 is configured to be slightly recessed, which facilitates positioning and assembly during initial fitting.

As shown in FIG. 6, the axial lengths of the front groove segment 131 and the inclined guiding surface 133 are both greater than the axial length of the rear groove segment 132. This provides a longer stroke in the pressing trigger phase. In case of accidental pressing without reaching near the limit position, the second sheath 3 rebounds under spring force and the entire injection needle can still be reused, realizing an accidental pressing prevention function.

Specifically, the entire second sheath 3 is made of transparent material or provided with a transparent portion. After use, when the second sheath 3 and transmission ring 6 extend and reset under spring force and the first bump 61 reaches the end position B of the inclined guiding segment 332, the transmission ring 6 can be seen protruding from the first opening 21 of the first sheath 2 through the transparent portion of the second sheath 3 (as shown in FIG. 17), distinguishing used needles from unused ones.

The present embodiment has the following advantages:
1. After the second sheath 3 resets upon single use, the second bump 2 of the transmission ring rotates to face the locking surface 134. The locking surface 134 blocks the second bump 62 to prevent rearward movement of the transmission ring 6, and the first bump 61 of the transmission ring 6 blocks the second sheath 3 accordingly. The second sheath 3 can't move rearward again and permanently covers the tip of the needle tube 11 to realize the anti-reuse protection function;
2. Excellent operational stability and high safety;
3. Simple overall structure;
4. Easy assembly, supporting automated production;
5. Comfortable hand feel during operation.

The above embodiment is only used to illustrate the technical concept and features of the present invention, so that those skilled in the art can understand and implement the present invention, and shall not be construed as limiting the protection scope of the present invention. All equivalent variations and modifications made in accordance with the spirit of the present invention shall fall within the protection scope thereof.

## Claims

1. A safety injection needle, comprising a needle hub, a first sheath, a second sheath and a spring; an axially extending needle tube is arranged on the needle hub; the first sheath is sleeved on the needle hub and fixedly connected to the rear end thereof, and a first opening is formed at the front end of the first sheath; the needle tube of the needle hub extends out from the first opening, and an accommodating space is formed between the first sheath and the needle hub; the rear end of the second sheath is arranged inside the accommodating space, the front end of the second sheath is provided with a second opening and extends out from the first opening, and the second sheath is axially slidably connected relative to the first sheath; **characterized in that**:
A transmission ring is arranged between the inner side of the rear port of the second sheath and the needle hub; at least one first bump is circumferentially arranged on the outer wall of the transmission ring, and at least one second bump is circumferentially arranged on the inner wall of the transmission ring;
A blocking guiding surface corresponding to the first bump is formed on the inner wall of the second sheath; the blocking guiding surface includes a flat guiding segment and an inclined guiding segment; one end of the flat guiding segment is defined as an initial position, the other end thereof is connected to one end of the inclined guiding segment, the inclined guiding segment inclines toward the front end of the second sheath, and the other end of the inclined guiding segment is defined as an end position;
An axially extending guiding groove corresponding to the second bump is formed on the outer circumference of the needle hub; the guiding groove includes a front groove segment and a rear groove segment; the front groove segment has a larger circumferential dimension than the rear groove segment; one side wall of the front groove segment is connected to the same side wall of the rear groove segment via an inclined guiding surface, and a locking surface facing forward is formed at the front end of the other side of the front groove segment;
The spring is sleeved on the needle hub; a spring mounting surface abutting the rear end of the spring is formed on the needle hub, and the front end of the spring abuts against the transmission ring;
In the initial state before use, the first bump of the transmission ring is located at the initial position of the blocking guiding surface, and the second bump directly faces the side wall of the front groove segment close to the inclined guiding surface; during use, the second sheath moves rearward under external force, the blocking guiding surface cooperates with the first bump to push the transmission ring to move rearward, the second bump slides into the front groove segment, and the cooperation between the second bump and the inclined guiding surface drives the transmission ring to rotate circumferentially; when the second sheath moves rearward to the limit position, the first bump slides onto the inclined guiding segment, and the second bump slides to the rear end of the rear groove segment; after use, the second sheath and the transmission ring extend and reset under the spring force, the second bump slides to the front end of the front groove segment, and under the sliding cooperation between the first bump and the inclined guiding segment, the second bump rotates to a position opposite the locking surface, so the second sheath cannot retract rearward again due to the blocking of the locking surface.

2. The safety injection needle according to Claim 1, **characterized in that** the locking surface corresponds to the end position of the inclined guiding segment, and the circumferential width of the locking surface is greater than the circumferential width of the end position of the inclined guiding segment.

3. The safety injection needle according to Claim 2, **characterized in that** a forward protruding portion is formed at the junction of the locking surface and the front groove segment to make the locking surface concave.

4. The safety injection needle according to Claim 1, **characterized in that** an axial transition surface is arranged between the flat guiding segment and the inclined guiding segment of the blocking guiding surface, so that the first bump abuts against the axial transition surface when the second sheath moves rearward to the limit position.

5. The safety injection needle according to Claim 1, **characterized in that** a circumferential annular protrusion is formed on the rear end of the needle hub, a clamping portion is correspondingly formed on the inner wall of the rear end of the first sheath, and the first sheath and the needle hub are connected via clamping fit between the clamping portion and the annular protrusion.

6. The safety injection needle according to Claim 1, **characterized in that** the second sheath is provided with a transparent portion; after use, when the second sheath and the transmission ring extend and reset under spring force and the first bump reaches the end position of the inclined guiding segment, the transmission ring can be seen protruding from the first opening of the first sheath through the transparent portion of the second sheath.

7. The safety injection needle according to Claim 1, **characterized in that** the axial lengths of the front groove segment and the inclined guiding surface are both greater than the axial length of the rear groove segment.
